# EUROPEAN PATENT SPECIFICATION

(11) **EP 3 331 903 B1**
(45) Date of publication and mention of the grant of the patent: **02.10.2019**
(21) Application number: 16751259.9
(22) Date of filing: 05.08.2016
(51) Int. Cl.: C07K 14/745

(54) **HETEROMERS COMPRISING ANTIBODY DOMAIN FUSION PROTEINS**
HETEROMERE MIT ANTIKÖRPERDOMÄNEN-FUSIONSPROTEINEN
HÉTÉROMÈRES COMPRENANT DES PROTÉINES FUSIONNÉES À UN DOMAINE D'ANTICORPS

(30) Priority: 06.08.2015 LU 92791; 07.08.2015 EP 15180252
(43) Date of publication of application: 13.06.2018
(73) Proprietor: Glycotope GmbH, 13125 Berlin (DE)
(72) Inventor: GOLETZ, Steffen, 13125 Berlin (DE); JAHN, Doreen, 13125 Berlin (DE)
(74) Representative: Hemsath, Lars
(86) International application number: PCT/EP2016/068730
(87) International publication number: WO 2017/021528

(56) References cited:
- WO-A1-99/37791
- WO-A1-2012/170289
- C. ROZAN ET AL: "Single-Domain Antibody-Based and Linker-Free Bispecific Antibodies Targeting Fc RIII Induce Potent Antitumor Activity without Recruiting Regulatory T Cells", MOLECULAR CANCER THERAPEUTICS, vol. 12, no. 8, 1 August 2013 (2013-08-01) , pages 1481-1491, XP055156453, ISSN: 1535-7163, DOI: 10.1158/1535-7163.MCT-12-1012
- MULLER K M ET AL: "The first constant domain (CH1 and CL) of an antibody used as heterodimerization domain for bispecific miniantibodies", FEBS LETTERS, ELSEVIER, AMSTERDAM, NL, vol. 422, no. 2, 30 January 1998 (1998-01-30), pages 259-264, XP004261818, ISSN: 0014-5793, DOI: 10.1016/S0014-5793(98)00021-0
- SCHOONJANS R ET AL: "A new model for intermediate molecular weight recombinant bispecific and trispecific antibodies by efficient heterodimerization of single chain variable domains through fusion to a Fab-chain", BIOMOLECULAR ENGINEERING, ELSEVIER, NEW YORK, NY, US, vol. 17, no. 6, 1 June 2001 (2001-06-01), pages 193-202, XP002207880, ISSN: 1389-0344, DOI: 10.1016/S1389-0344(01)00066-1
- D. GAILANI: "Model for a factor IX activation complex on blood platelets: dimeric conformation of factor XIa is essential", BLOOD, vol. 97, no. 10, 15 May 2001 (2001-05-15), pages 3117-3122, XP055059250, ISSN: 0006-4971, DOI: 10.1182/blood.V97.10.3117
- RAO BENQIANG ET AL: "A recombinant adenovirus vector encoding the light chain of human coagulation factor VII and IgG1 Fc fragment to targeting tissue factor for colorectal cancer immunotherapy in the mouse model", JOURNAL OF CANCER RESEARCH AND CLINICAL ONCOLOGY,, vol. 139, no. 6, 1 June 2013 (2013-06-01), pages 1015-1023, XP002745724,

## Description

### FIELD OF THE INVENTION

The present invention pertains to heteromers of fusion polypeptides, wherein dimerization is provided by two antibody domains. In particular, the CH1 domain of an antibody is fused to a first portion of a protein and the CL domain of an antibody is fused to a second portion of said protein. By dimerization via the CH1 and CL domains, the protein is formed by the two portions.

### BACKGROUND OF THE INVENTION

Blood coagulation is an important natural process for sealing wounds in the human or animal body. It consists of a complex interaction of various blood components which eventually result in a fibrin clot. The blood components, coagulation factors, which are involved in the coagulation cascade are proenzymes, enzymatically inactive proteins which are converted to protease enzymes by proteolytic cleavage. Thereby a coagulation cascade is formed wherein one coagulation factor cleaves and activates the next coagulation factor. Coagulation factors which have undergone such a conversion are generally referred to as "activated factors" and are designated by the addition of a lowercase postscript "a" (e.g., factor VIIa). The coagulation factors are generally serine proteases which act by cleaving downstream proteins. Examples of these serine protease factors are thrombin and factors VII, IX, X, XI and XII.

Factor VII is a single-chain glycoprotein with a molecular weight of about 50 kDa Activation of factor VII to VIIa may be catalyzed by several different plasma proteases, such as factor XIIa. Activation of factor VII results in the formation of two polypeptide chains, a heavy chain of 28 kDa and a light chain of 17 kDa held together by at least one disulfide bond. Factor IX is also a single-chain precursor of 57 kDa and is converted to an active serine protease (factor IXa) upon cleavage by Factor XIa. Factor IXa consists of a 16 kDa light chain and a 29 kDa heavy chain.

Various coagulation factors such as in particular factors VII, VIII and IX are used in medicine in the treatment of hemophilia or to prevent uncontrollable hemorrhage. In therapy, the proenzyme may be administered which is then activated in the body by the patient's coagulation cascade. Since the inactive factor generally is only partially activated in the patient's body and the rate of activation may vary in different patients, dosing of this drug involves uncertainties. In another approach, the factor is artificially activated prior to administration to the patient. However, this approach requires a specific activation step and is difficult to control because the factor may also be degraded during the activation step. Similar problems are also associated with other therapeutic proteins which - in their active form - consist of two separate portions or subunits.

WO9937791 discloses in general the use of Fab antibody fragments as heterodimerization scaffolds. WO2012170289 describes heterodimeric FVIII constructs comprising two separate polypeptide chains consisting each of various combinations of FVIII domains.

Therefore, it is the object of the present invention to provide means for the production of dimeric or multimeric proteins with improved properties.

### SUMMARY OF THE INVENTION

The present inventors found that proteins consisting of two or more polypeptide chains (subunits) such as activated coagulation factors, in particular factor VIIa, can be produced by recombinant expression using two fusion polypeptide constructs, wherein the first portion is fused to the CH1 domain of an antibody and the second portion is fused to the CL domain of an antibody. For proteins which are normally produced as inactive single chain protein and which have to be activated by cleavage into two separate polypeptide chains, such as the blood coagulation factors, the production of the protein already in the active form comprising two separate polypeptide chains is especially advantageous because the activation step necessary for the single chain protein - either artificially or in the patient's body - is avoided.

The production as CH1/CL fusion polypeptides further increases stability and circulation half-life of the proteins. The half-life of the blood coagulation factor in the circulation of the patient is a critical factor in the treatment. In particular in the treatment of congenital diseases such as hemophilia, the blood coagulation factor is continuously administered to the patient. Therapeutics with prolonged half-life hence reduce the amount of drug and the number of administrations needed. Therefore, the use of CH1-CL heteromers is particularly advantageous for blood coagulation factors such as Factor VII.

Hence, in a first aspect, the present invention provides a heteromer comprising a first fusion polypeptide and a second fusion polypeptide, wherein the first fusion polypeptide comprises a first portion of a protein of interest fused to a CH1 domain of an antibody, and the second fusion polypeptide comprises a second portion of the protein of interest fused to a CL domain of an antibody; wherein the protein of interest is an activated blood coagulation factor. In the heteromer, the first portion and the second portion congregate so as to form the protein of interest. One specific example is factor VIIa, wherein the first portion is derived from the light chain of factor VIIa and the second portion is derived from the heavy chain of factor VIIa.

A heteromer comprising a first fusion polypeptide and a second fusion polypeptide, wherein the first fusion polypeptide comprises a first protein of interest fused to a CH1 domain of an antibody, and the second fusion polypeptide comprises a second protein of interest fused to a CL domain of an antibody is also described. In the heteromer, in particular the first protein and the second protein congregate so as to form a protein complex.

In a second aspect, the present invention provides a nucleic acid encoding the first fusion polypeptide and/or the second fusion polypeptide. In a third aspect, the present invention provides a cell comprising said nucleic acid.

In a fourth aspect, the present invention provides a method of producing the heteromer according to the first or second aspect, wherein the first fusion polypeptide and the second fusion polypeptide are expressed in mammalian cells.

Other objects, features, advantages and aspects of the present invention will become apparent to those skilled in the art from the following description and appended claims.

### DEFINITIONS

As used herein, the following expressions are generally intended to preferably have the meanings as set forth below, except to the extent that the context in which they are used indicates otherwise.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of".

As used herein, the term "polypeptide" refers to a molecular chain of amino acids. A polypeptide can contain any of the naturally occurring amino acids as well as artificial amino acids and can be of biologic or synthetic origin. A polypeptide may be modified, naturally (post-translational modifications) or synthetically, by e.g. glycosylation, amidation, carboxylation, hydroxylation and/or phosphorylation. A polypeptide comprises at least two amino acids, but does not have to be of any specific length; this term does not include any size restrictions. Preferably, a polypeptide comprises at least 50 amino acids, more preferably at least 100 amino acids, most preferably at least 150 amino acids.

The term "antibody" in particular refers to a protein comprising at least two heavy chains and two light chains connected by disulfide bonds. Each heavy chain is comprised of a heavy chain variable region (VH) and a heavy chain constant region (CH). Each light chain is comprised of a light chain variable region (VL) and a light chain constant region (CL). The heavy chain-constant region comprises three or - in the case of antibodies of the IgM- or IgE-type - four heavy chain-constant domains (CH1, CH2, CH3 and CH4) wherein the first constant domain CH1 is adjacent to the variable region and may be connected to the second constant domain CH2 by a hinge region. The light chain-constant region consists only of one constant domain (CL).

In particular, the antibody may be of any isotype such as IgA, IgD, IgE, IgG or IgM, including any subclass such as IgG1, IgG2, IgG3, IgG4, IgA1 or IgA2. Preferably, the antibody is an IgG antibody, more preferably an IgG1- or IgG2-antibody, in particular an IgG1-antibody. The heavy chain constant regions may be of any type such as γ-, δ-, α-, µ- or ε-type heavy chains. Preferably, the heavy chain of the antibody is a γ-chain. Furthermore, the light chain constant region may also be of any type such as κ- or λ-type light chains. Preferably, the light chain of the antibody is a κ-chain. Preferably the antibody is a full length antibody which in the case of IgG antibodies comprises two full length heavy chains and two full length light chains. In specific embodiments, the term "antibody" refers to naturally occurring antibodies, in particular naturally occurring human antibodies.

A target amino acid sequence is "derived" from a reference amino acid sequence if the target amino acid sequence shares a homology or identity over its entire length with a corresponding part of the reference amino acid sequence of at least 75%, more preferably at least 80%, at least 85%, at least 90%, at least 93%, at least 95% or at least 97%. For example, if a CH1 domain of a fusion polypeptide is derived from the CH1 domain of a particular antibody, then the amino acid of the CH1 domain of the fusion polypeptide shares a homology or identity over its entire length with the CH1 domain of the antibody of at least 75%, more preferably at least 80%, at least 85%, at least 90%, at least 93%, at least 95% or at least 97%. The same is true, for example, for a CL domain of a fusion polypeptide derived from the CL domain of a specific antibody. In particular embodiments, a target amino acid sequence which is "derived" from a reference amino acid sequence is 100% homologous, or in particular 100% identical, over its entire length with a corresponding part of the reference amino acid sequence. A "homology" or "identity" of an amino acid sequence or nucleotide sequence is preferably determined according to the invention over the entire length of the reference sequence or over the entire length of the corresponding part of the reference sequence which corresponds to the sequence which homology or identity is defined.

The term "a part" of a protein or polypeptide in particular refers to a functional part which exhibits one or more of the properties of the protein or polypeptide. In particular, in case the protein or polypeptide has a specific enzymatic activity or binding ability, the part of said protein or polypeptide also has the specific enzymatic activity or binding ability, optionally in a lesser degree or level. The part of the protein or polypeptide in particular encompasses at least 20%, especially at least 30%, at least 40%, at least 50%, at least 60%, at least 70%, at least 80% or at least 90% of the amino acid sequence of the protein or polypeptide.

The term "patient" in particular refers to an animal or human, especially to a mammal. In specific embodiments, the patient is a human being.

The term "pharmaceutical composition" and similar terms particularly refer to a composition suitable for administering to a human or animal, i.e., a composition containing components which are pharmaceutically acceptable. Preferably, a pharmaceutical composition comprises an active compound or a salt or prodrug thereof together with a carrier, diluent or pharmaceutical excipient such as buffer, preservative and tonicity modifier.

### DETAILED DESCRIPTION OF THE INVENTION

In a first aspect, the present invention provides a heteromer comprising a first fusion polypeptide and a second fusion polypeptide, wherein the first fusion polypeptide comprises a first portion of a protein of interest fused to a CH1 domain of an antibody, and the second fusion polypeptide comprises a second portion of the protein of interest fused to a CL domain of an antibody; wherein the protein of interest is an activated blood coagulation factor.

The heteromer is used to provide a protein of interest which comprises two portions. Each portion of the protein is formed by a separate polypeptide chain. Via assembly of the portions, the protein of interest is obtained, in particular in a functional form. According to the present invention, one of the portions of the protein is fused to a CH1 domain of an antibody and another portion is fused to a CL domain of an antibody. For purpose of the present invention only, the portion fused to the CH1 domain is designated "first portion" and the portion fused to the CL domain is designated "second portion". The designators "first" and "second" are only used for distinguishing the portions, but shall not indicate any order of the portions. In certain embodiments, the heteromer consists of the first fusion polypeptide and the second fusion polypeptide. In these embodiments, the heteromer is a heterodimer.

The CH1 domain and the CL domain of an antibody have a natural affinity and bind to each other when brought into contact. The binding between CH1 and CL may even further be strengthened by an intermolecular disulfide bridge which forms between a cysteine of the CH1 domain and a cysteine of the CL domain. Therefore, in the constructs described herein the binding of CH1 and CL support the assembly of the protein of interest by bringing the first and second portion in close proximity. Thereby, even proteins which portions do not readily assemble can be obtained. Hence, in the heteromer the first fusion polypeptide and the second fusion polypeptide in particular bind to each other, especially via non-covalent and/or covalent binding.

Furthermore, the CH1 and CL domains are immunoglobulin domains which have the immunoglobulin fold. Immunoglobulin domains and in particular the CH1 and CL domains are highly stable structures which readily adopt their correct three-dimensional fold and may further be stabilized by an intramolecular disulfide bridge. Furthermore, these domains have a high solubility in aqueous solvents and are easily produced by expression cell lines in high yields. Fusing the portions of a protein of interest to the CH1 or CL domain of an antibody in certain embodiments increases the production rate, yield and/or stability of the portions. It may even be possible to produce each portion separately as fusion polypeptide with either CH1 or CL and the combine the fusion polypeptides to form the protein of interest.

Moreover, the addition of the CH1 domain and the CL domain to the portions of the protein of interest increases the total size of the protein. This is in particular advantageous for therapeutic proteins. Small proteins are removed from the circulation via renal filtration. However, proteins having a molecular weight above a certain threshold are filtered from the blood stream at a markedly lower rate. Each immunoglobulin domain has a molecular weight of about 10-12.5 kDa. In the heteromer described herein the molecular weight of the protein of interest therefore is increase at least by 20 kDa and may even be further increased in embodiments wherein the fusion polypeptides additionally comprise linkers and/or further fusion partners.

For forming the protein of interest using the fusion polypeptides described herein, the CH1 domain and the CL domain are advantageously fused to those termini of the portions which are in close proximity in the assembled protein of interest. However, using suitable linker sequences between the portion and the CH1 or CL domain, these domains can also be fused to termini of the portions which are more remote from each other in the assembled protein of interest.

The term "CH1/CL domain" as used herein refers to each of the CH1 domain and the CL domain separately as well as to both the CH1 domain and the CL domain. Hence, if a feature is attributed to the CH1/CL domain, then said feature may apply to only the CH1 domain or to only the CL domain or to both the CH1 domain and the CL domain. The terms "CH1 domain" and "CL domain" further also encompass domains which are derived from the respective naturally occurring domain and/or functional parts of the respective domains which are sufficient to facilitate binding of the two domains with each other.

In the first fusion polypeptide, the CH1 domain may be fused to the C terminus or to the N terminus of the first portion. Likewise, in the second fusion polypeptide, the CL domain may be fused to the C terminus or to the N terminus of the second portion. In certain embodiments, the CH1 domain is fused to the C terminus of the first portion. In further embodiments, the CL domain is fused to the C terminus of the second portion. In particular embodiments, the CH1 domain is fused to the C terminus of the first portion and the CL domain is fused to the C terminus of the second portion.

The CH1/CL domain may be fused to the portion either directly or via a linker. A direct fusion refers to polypeptides wherein the sequence of the CH1/CL domain directly follows or precedes the sequence of the portion without any intermediate amino acids between these two sequences. A fusion via a linker refers to polypeptides wherein one or more amino acids are present between the sequence of the CH1/CL domain and the sequence of the portion. These one or more amino acids form the linker between the CH1/CL domain and the portion. In these embodiments, the first fusion polypeptide comprises the first portion, a linker and the CH1 domain and/or the second fusion polypeptide comprises the second portion, a linker and the CL domain.

The linker may in principle have any number of amino acids and any amino acid sequence which are suitable for linking the CH1/CL domain an the portion. In certain embodiments, the linker between the CH1/CL domain and the portion comprises at least 3, preferably at least 5, at least 8, at least 10, at least 15 or at least 20 amino acids. In further embodiments, the linker between the CH1/CL domain and the portion comprises 50 or less, preferably 45 or less, 40 or less, 35 or less, 30 or less, 25 or less or 20 or less amino acids. In particular, the inker between the CH1/CL domain and the portion comprises from 5 to 20 amino acids. In specific embodiments, the linker consists of glycine and serine residues. Glycine and serine may be present in the linker in a ratio of 2 to 1, 3 to 1, 4 to 1 or 5 to 1 (number of glycine residues to number of serine residues). For example, the linker may comprise a sequence of four glycine residues followed by one serine residue, and in particular 1, 2, 3, 4, 5 or 6 repeats of this sequence. Specific examples are linkers consisting of 2 repeats of the amino acid sequence GGGGS (SEQ ID NO: 1), 4 repeats of the amino acid sequence GGGGS

(SEQ ID NO: 1) and 6 repeats of the amino acid sequence GGGGS (SEQ ID NO: 1). Especially linkers consisting of 6 repeats of the amino acid sequence GGGGS (SEQ ID NO: 1) may be used. In other embodiments the linker comprises sequences which show no or only minor immunogenic potential in humans, preferably sequences which are human sequences or naturally occurring sequences. In a further preferred embodiment the linkers and the adjacent amino acids show no or only minor immunogenic potential.

In certain embodiments, the first and/or the second fusion polypeptide comprise a further fusion partner. The fusion partner may be present N terminally to the CH1/CL domain and the portion of the protein of interest, C terminally to the CH1/CL domain and the portion of the protein of interest or between the CH1/CL domain and the portion of the protein of interest. In certain embodiments, the CH1/CL domain is present between the portion of the protein of interest and the further fusion partner. The further fusion partner may be fused to the CH1/CL domain and/or the portion of the protein of interest directly or indirectly, in particular using a linker as described above. In certain embodiments, the first fusion polypeptide comprises a further fusion partner.

The further fusion partner may be any peptide, polypeptide or protein, including, for example, peptides, polypeptide and proteins which increase the half-life of the protein of interest in the human or animal circulation, peptides, polypeptide and proteins which provide a detectable label, and peptides, polypeptide and proteins which improve the purification and/or stability of the fusion polypeptide. In specific embodiments, the further fusion partner increase the half-life of the protein of interest in the human or animal circulation. For example, the further fusion partner may be a protein or polypeptide with a size which is large enough to exceed the threshold value of renal clearing, in particular a molecular weight of at least 40 kDa, especially at least 50 kDa, at least 60 kDa or at least 70 kDa. In further embodiments, the further fusion partner may be capable of binding to receptors which prolongs the half-life, such as being able to bind to the FcRn. The further fusion partner may in particular be selected from the group consisting of albumin, the hinge, CH2 and/or CH3 portion of an antibody, elastin-like polypeptides (ELP), albumin binding domains, His-tag and glutathione-S-transferase (GST) tag, or a part thereof, or the further fusion partner may be derived from one of these proteins.

In certain embodiments, the further fusion partner is serum albumin, in particular human serum albumin, or a fusion partner derived therefrom and/or a part thereof which is capable of elongating the circulation half-life of the heteromer. The serum albumin especially is present in the first fusion polypeptide. In specific embodiments, the first fusion polypeptide comprises, consecutively from the N terminus to the C terminus, the portion of the protein of interest, optionally a linker, the CH1 domain, optionally a linker, and the further fusion partner which is derived from human serum albumin or a part thereof. In these embodiments, the second fusion polypeptide in particular comprises, consecutively from the N terminus to the C terminus, the portion of the protein of interest, optionally a linker, and the CL domain.

In further embodiments, the further fusion partner comprises the hinge, CH2 and/or CH3 portion of an antibody, in particular the hinge, CH2 and CH3 portion of an antibody, or a functional part thereof, or it is derived therefrom. This further fusion partner in particular is present in the first fusion polypeptide. In specific embodiments, the first fusion polypeptide comprises, consecutively from the N terminus to the C terminus, the portion of the protein of interest, optionally a linker, the CH1 domain, optionally a linker, and the further fusion partner which is derived from the hinge, CH2 and CH3 portion of an antibody or a part thereof. In these embodiments, the second fusion polypeptide in particular comprises, consecutively from the N terminus to the C terminus, the portion of the protein of interest, optionally a linker, and the CL domain. Furthermore, in these embodiments, the heteromer in particular comprises a further polypeptide chain which comprises a CH2 and a CH3 domain of an antibody and binds to the CH2 and CH3 domains of the further fusion partner. Specifically, the CH2 and CH3 domains in these embodiments comprise mutations which reduce the binding affinity of CH2 and CH3 domains with the same mutations. Thereby, the CH2 and CH3 domains of the further fusion partner having a first set of mutations bind with a much higher affinity to the CH2 and CH3 domains of the further polypeptide chain than to the CH2 and CH3 domains of another further fusion partner. Similarly, the CH2 and CH3 domains of the further polypeptide chain having a second set of mutations bind with a much higher affinity to the CH2 and CH3 domains of the further fusion partner than to the CH2 and CH3 domains of another further polypeptide chain. In certain embodiments, one or more disulfide bridges are formed between the CH2 and CH3 domains of the further fusion partner and the CH2 and CH3 domains of the further polypeptide chain.

In specific embodiments, the further fusion partner does not comprise a hinge region, a CH2 domain and a CH3 domain of an antibody, in particular not a CH2 domain and a CH3 domain of an antibody, especially not a CH2 domain or a CH3 domain of an antibody.

In further embodiments, the first fusion polypeptide does not comprise a hinge region, a CH2 domain and a CH3 domain of an antibody, in particular not a CH2 domain and a CH3 domain of an antibody, especially not a CH2 domain or a CH3 domain of an antibody. In particular, the first fusion polypeptide does not comprise all of a hinge region, a CH2 domain and a CH3 domain of an antibody, and especially does not comprise any one of a CH2 domain and a CH3 domain of an antibody, particularly any one of a hinge region, a CH2 domain and a CH3 domain of an antibody.

The CH1 domain and the CL domain are preferably derived from the same species, in particular a human CH1 domain and a human CL domain are used. A CH1 domain from any antibody may be used, such as a CH1 domain of an IgG, IgM, IgA, IgD or IgE antibody. In particular, the CH1 domain is derived from an IgG antibody, especially from an IgG1 antibody. The CH1 domain may be derived from a naturally occurring γ-, δ-, α-, µ- or ε-type heavy chain, in particular from a γ-type heavy chain, especially a human heavy chain such as a human γ-type heavy chain. The CH1 domain in particular may comprise the amino acid sequence of SEQ ID NO: 2 or an amino acid sequence which is at least 80%, preferably 90% or 95% homologous or identical to SEQ ID NO: 2 over the entire length of SEQ ID NO: 2. In specific embodiments, the CH1 domain consists of an amino acid sequence which is at least 90% identical to SEQ ID NO: 2 over the entire length of SEQ ID NO: 2. In another embodiment, the CH1 domain consists of an amino acid sequence of SEQ ID NO: 2. Furthermore, in certain embodiments, the CH1 domain additionally comprises an amino acid sequence comprising at least one cysteine residue at its C-terminus. This additional amino acid sequence in particular has a length of 10 or less, in particular 7 or less or 5 or less, residues and especially consists of the sequence of SEQ ID NO: 3 or a part thereof, in particular amino acids 1-5 or 1-7 of SEQ ID NO: 3, or a single cysteine residue.

The CL domain of any antibody may be used. The CL domain may be derived from a naturally occurring κ- or A-type light chain, in particular from a κ-type light chain, especially a human light chain such as a human κ-type light chain. The CL domain in particular may comprise the amino acid sequence of SEQ ID NO: 4, 5 or 6 or an amino acid sequence which is at least 80%, preferably 90% or 95% homologous or identical to SEQ ID NO: 4, 5 or 6 over the entire length of SEQ ID NO: 4, 5 or 6, respectively. In specific embodiments, the CL domain consists of an amino acid sequence which is at least 90% identical to SEQ ID NO: 4, 5 or 6 over the entire length of SEQ ID NO: 4, 5 or 6, respectively. In another embodiment, the CL domain consists of an amino acid sequence of SEQ ID NO: 4, 5 or 6.

The CH1 domain and the CL domain in particular are capable of specifically binding to each other. In certain embodiments, the CH1 domain and the CL domain form an intermolecular disulfide bridge in the heteromer described herein. This disulfide bridge may involve the cysteine residue of the additional amino acid sequence comprising at least one cysteine residue at its C-terminus of the CH1 domain, in particular the cysteine residue at amino acid position 5 in SEQ ID NO: 3 or a cysteine residue in the amino acid sequence of the CH1 domain corresponding thereto, and/or the cysteine residue at amino acid position 106 or 105 in SEQ ID NO: 4, 5 or 6, respectively, or a cysteine residue in the amino acid sequence of the CL domain corresponding thereto.

In general, a protein of interest may be any protein comprising two or more portions, in particular any protein consisting of two portions. In certain embodiments, the protein of interest is a naturally occurring protein or is derived therefrom and/or is a part thereof. The term "portion" in this respect in particular refers to a distinct polypeptide and each portion is formed by a separate polypeptide. In certain embodiments, the "portions" of the protein of interest are subunits of the protein of interest. The portions of the protein may be different or identical, preferably different from each other. If the protein of interest comprises more than two portions, the at least one portion is present in the first fusion polypeptide and at least another portion is present in the second fusion polypeptide. The further portions of the protein of interest in particular are also part of the heteromer, and may be part of one or both of the fusion polypeptides or may be present in the heteromer not as part of any of the fusion polypeptides.

Suitable examples of a protein of interest are activated blood coagulation factors and gonadotropins. The protein of interest may be a naturally occurring protein or an artificial protein. Furthermore, the portions may comprise or consist of the natural amino acid sequences of said protein or may be engineered. This includes amino acid sequences which are derived from the natural amino acid sequences and truncated version of the natural amino acid sequences or the amino acid sequences derived therefrom. In specific embodiments, the protein of interest present in the heteromer exhibits one or more of the properties, in particular biological and/or enzymatic activities and/or binding affinities and/or specificities, of the natural protein from which it is derived. This includes embodiments wherein the properties of the protein of interest in the heteromer are lower than in the natural protein from which it is derived. For example, the protein of interest has at least 1%, in particular at least 10% or at least 25% of the biological or enzymatic activity or binding affinity or specificity of the natural protein.

According to the invention, the protein of interest is an activated blood coagulation factor. Blood coagulation factors are generally expressed as inactive precursor proteins which are activated by cleavage of the polypeptide strain via enzymatic digestion. The activated blood coagulation factor can then act as serine protease and cleave a downstream target protein which in turn is activated thereby. The two separate polypeptide chains of an activated blood coagulation factor, i.e. the light chain and the heavy chain, may also be recombinantly expressed. With this approach, activation by an upstream factor is not necessary. In particular, the protein may be selected from the group consisting of fibrin, thrombin, factor Va, factor VIIa, factor VIIIa, factor IXa, factor Xa, factor XIa, factor XIIa and factor XIIIa. In certain embodiments wherein the protein of interest is a blood coagulation factor or a part thereof or derived therefrom, the heteromer in particular has the serine protease enzymatic activity of the respective blood coagulation factor.

In a specific embodiment, the first portion is the light chain of the activated blood coagulation factor or a part thereof and the second portion is the heavy chain of the activated blood coagulation factor or a part thereof. For example, the protein is factor VIIa and the first portion is the light chain of factor VIIa or a part thereof and the second portion is the heavy chain of factor VIIa or a part thereof. In an alternative embodiment, the first portion is the heavy chain of the activated blood coagulation factor or a part thereof and the second portion is the light chain of the activated blood coagulation factor or a part thereof. For example, the protein is factor VIIa and the first portion is the heavy chain of factor VIIa or a part thereof and the second portion is the light chain of factor VIIa or a part thereof. The light chain of factor VIIa in particular comprises the amino acid sequence of positions 61 to 212 of SEQ ID NO: 7 or is derived therefrom and the heavy chain of factor VIIa in particular comprises the amino acid sequence of positions 213 to 466 of SEQ ID NO: 7 or is derived therefrom.

Hence, in specific embodiments a heteromer is provided, wherein the heteromer comprises a first fusion polypeptide and a second fusion polypeptide, wherein the first fusion polypeptide comprises the light chain of factor VIIa fused to a CH1 domain of an antibody, optionally further fused to human serum albumin, and the second fusion polypeptide comprises the heavy chain of factor VIIa fused to a CL domain of an antibody. This includes embodiments wherein the heterodimer comprises functional parts of the respective elements, such as functional parts of the light chain and/or heavy chain of factor VIIa which are capable of providing the enzymatic activity of factor VIIa.

Also described is a protein of interest which is a gonadotropin, in particular a gonadotropin selected from the group consisting of follicle stimulating hormone (FSH), luteinizing hormone (LH), human chorionic gonadotropin (hCG) and thyroid stimulating hormone (TSH). The first portion may be the alpha chain of the gonadotropin and the second portion may be the beta chain of the gonadotropin. Alternatively, the first portion may be the beta chain of the gonadotropin and the second portion may be the alpha chain of the gonadotropin.

In specific embodiments, the protein of interest is not an antibody. In particular, the first portion and/or the second portion is not a variable region of an antibody. Specifically, the first portion is not a heavy chain variable region and/or the second portion is not a light chain variable region.

The heteromerization technology described herein may also be used to provide complex formation of two proteins of interest. Herein, the two portions present in the heteromer according to the first aspect are replaced by proteins, i.e. polypeptides which are not portions of the same protein. Hence, a heteromer comprising a first fusion polypeptide and a second fusion polypeptide is provided, wherein the first fusion polypeptide comprises a first protein of interest fused to a CH1 domain of an antibody, and the second fusion polypeptide comprises a second protein of interest fused to a CL domain of an antibody. In the heteromer, in particular the first protein and the second protein congregate so as to form a protein complex. The features, embodiments and examples described herein for the heteromer comprising a first and a second portion of a protein of interest likewise apply to the heteromer comprising a first protein of interest and a second protein of interest.

The first protein of interest and the second protein of interest may be any polypeptide. The first and the second protein of interest for example may be naturally occurring proteins or artificial proteins. They may comprise or consist of the natural amino acid sequences of said proteins or may be engineered. This includes amino acid sequences which are derived from the natural amino acid sequences and truncated version of the natural amino acid sequences or the amino acid sequences derived therefrom. In specific embodiments, the first and/or the second protein of interest present in the heteromer exhibit one or more of the properties, in particular biological and/or enzymatic activities and/or binding affinities and/or specificities, of the natural proteins from which they are derived. This includes embodiments wherein the properties of the first and/or the second protein of interest in the heteromer are lower than in the natural proteins from which they are derived. For example, the first and/or the second protein of interest have at least 1%, in particular at least 10% or at least 25% of the biological or enzymatic activity or binding affinity or specificity of the natural proteins. In certain embodiments, the heteromer has a specific activity, especially enzymatic activity, which is not provided or provided only to a lesser extent by either of the first and second protein of interest when present alone. In particular, the heteromer when present in a suitable composition has a higher enzymatic activity than a corresponding composition comprising the same amount as the heteromer of each of the first and second protein of interest as free proteins - under the same conditions. In embodiments wherein one or both of the first and second protein of interest is a blood coagulation factor or a part thereof, the enzymatic activity in particular is a serine protease activity.

In certain embodiments, one or both of the first and second protein of interest are enzymes. In these embodiments, the heteromer preferably exhibits one or more of the enzymatic activities of the first and/or second protein of interest. In further embodiments, one of the first and second proteins of interest supports the stability, expression and/or three-dimensional structure, in particular the protein folding, of the other protein of interest. Here the one protein of interest may have chaperone-like properties and in particular may be derived from a chaperon or a functional part thereof. In further embodiments, one of the first and second proteins of interest is an enzyme and the other protein of interest modulates the enzymatic activity of said enzyme. Said modulation of the enzymatic activity in this respect may refer to a change, in particular increase, of the rate and/or the specificity of the enzymatic activity, the stability of the first protein portion, the binding affinity and/or specificity to the substrate, and/or the binding to a cofactor.

For example, the first protein of interest and/or the second protein of interest may be selected from the group consisting of blood coagulation factors, as proprotein or in activated form, including fibrin, thrombin, factor V, factor VII, factor VIII, factor IX, factor X, factor XI, factor XII, factor XIII and von Willebrand factor; gonadotropins, including FSH, LH, hCG, and TSH; and single chain antibody fragments such as scFv comprising the heavy chain and light chain variable region of a single arm of an antibody in one polypeptide; and parts thereof and proteins derived therefrom. In certain embodiments, the natural proteins from which the first and the second protein of interest are derived interact with each other in nature and/or are involved in the same biological pathway or reaction. In specific embodiments, one of the first and the second protein of interest is a blood coagulation factor or a part thereof. In particular, both the first and the second protein of interest are blood coagulation factors, for example the first protein of interest comprises factor VIIIa or a part thereof and the second protein of interest comprises von Willebrand factor or a part thereof, or vice versa.

In certain embodiments, the first protein of interest comprises factor VIIIa or a part thereof and the second protein of interest comprises von Willebrand factor or a part thereof. In alternative embodiments, the first protein of interest comprises von Willebrand factor or a part thereof and the second protein of interest comprises factor VIIIa or a part thereof. In these embodiments, factor VIIIa and von Willebrand factor in particular interact with each other. Especially, in these embodiments the heteromer has the biological activity of a factor VIIIa - von Willebrand factor complex, in particular the activity to accelerate the activation of factor X by activated factor IX in the coagulation cascade. The part of the von Willebrand factor especially comprises the D' domain and optionally the D3 domain, and/or in particular is capable of binding to factor Villa. In specific embodiments, the first and/or the second fusion polypeptide, in particular both fusion polypeptides, comprises a further fusion partner, especially a further fusion partner which elongates the circulation half-life of the heteromer such as albumin.

In specific embodiments, the first and/or the second protein of interest is not an antibody. In particular, the first and/or the second protein of interest is not a variable region of an antibody. Specifically, the first protein of interest is not a heavy chain variable region and/or the second first protein of interest is not a light chain variable region.

In a further aspect, the present invention provides a nucleic acid encoding the first fusion polypeptide and/or the second fusion polypeptide of the heteromer according to the invention. The nucleic acid sequence of the nucleic acid according to the invention may have any nucleotide sequence suitable for encoding the first fusion polypeptide and/or the second fusion polypeptide. However, preferably the nucleic acid sequence is at least partially adapted to the specific codon usage of the host cell or organism in which the nucleic acid according to the invention is to be expressed, in particular the human codon usage. The nucleic acid according to the invention may be double-stranded or single-stranded DNA or RNA, preferably double-stranded DNA such as cDNA or single-stranded RNA such as mRNA.

The nucleic acid may encode only the first fusion polypeptide or only the second fusion polypeptide or both the first fusion polypeptide and the second fusion polypeptide. In particular, the nucleic acid according to the invention may be a single nucleic acid molecule containing two coding regions wherein the first coding region encodes the first fusion polypeptide and the second coding region encodes the second fusion polypeptide, preferably separated by regulatory elements such as IRES elements in order to generate separate amino acid chains.

In addition to the coding regions encoding the fusion polypeptide, the nucleic acid according to the invention may also comprise further nucleic acid sequences or other modifications which, for example, may code for other proteins, may influence the transcription and/or translation of the coding region(s), may influence the stability or other physical or chemical properties of the nucleic acid, or may have no function at all.

In a further aspect, the present invention provides an expression cassette or vector comprising a nucleic acid according to the invention and a promoter operatively connected with said nucleic acid. In addition, the expression cassette or vector may comprise further elements, in particular elements which are capable of influencing and/or regulating the transcription and/or translation of the nucleic acid according to the invention, the amplification and/or reproduction of the expression cassette or vector, the integration of the expression cassette or vector into the genome of a host cell, and/or the copy number of the expression cassette or vector in a host cell. Suitable expression cassettes and vectors comprising respective expression cassettes for expressing antibodies are well known in the prior art and thus, need no further description here. In certain embodiments, a vector is provided which comprises one expression cassette comprising a nucleic acid according to the invention which codes for one of the fusion polypeptides. In further embodiments, a vector is provided which comprises one expression cassette comprising a nucleic acid according to the invention which codes for both of the fusion polypeptides. In even further embodiments, a vector is provided which comprises two expression cassette, each comprising a nucleic acid according to the invention which codes for one of the fusion polypeptides.

Furthermore, the present invention provides a host cell comprising the nucleic acid according to the invention or the expression cassette or vector according to the invention. The host cell may comprise one vector comprising one or two expression cassettes as described above. Furthermore, the host cell may comprise two vectors, one comprising an expression cassette coding for the first fusion polypeptide and the other comprising an expression cassette coding for the second fusion polypeptide. The host cell according to the invention may be any host cell. It may be an isolated cell or a cell comprised in a tissue. Preferably, the host cell is a cultured cell, in particular a primary cell or a cell of an established cell line, preferably a tumor-derived cell. Preferably, it is a bacterial cell such as E. coli, a yeast cell such as a Saccharomyces cell, in particular S. cerevisiae, an insect cell such as an Sf9 cell, or a mammalian cell, in particular a human cell such as a tumor-derived human cell, a hamster cell such as CHO, or a primate cell. Preferably, the codon usage in the coding region of the nucleic acid according to the invention and/or the promoter and the further elements of the expression cassette or vector are compatible with and, more preferably, optimized for the type of host cell used. Preferably, the heteromer according to the invention is produced by a host cell or cell line as described above. In certain embodiments, the first fusion polypeptide and the second fusion polypeptide are secreted by the host cells.

In a further aspect, the present invention provides a method of producing the heteromer described herein, wherein the first fusion polypeptide and the second fusion polypeptide are expressed in host cells, in particular in mammalian cells. The method in particular comprises the provision of host cells comprising nucleic acids encoding the first fusion polypeptide and the second fusion polypeptide. The nucleic acid may in particular be a nucleic acid as described above and the host cells may in particular be host cells as described above. In specific embodiments, the host cells comprise nucleic acids which encode the first fusion polypeptide as well as the second fusion polypeptide. In other embodiments, the host cells comprise nucleic acids which encode the first fusion polypeptide and further nucleic acids which encode the second fusion polypeptide. Hence, in certain embodiments the first fusion polypeptide and the second fusion polypeptide are co-expressed in the same host cell.

In another embodiment, the first fusion polypeptide and the second fusion polypeptide are expressed in different host cells. The heteromer according to the invention is formed after expression of the first and second fusion polypeptide by contacting the fusion polypeptides.

In specific embodiments, a method of producing the heteromer is provided, wherein the heteromer comprises a first fusion polypeptide and a second fusion polypeptide, wherein the first fusion polypeptide comprises the light chain of factor VIIa fused to a CH1 domain of an antibody, optionally further fused to human serum albumin, and the second fusion polypeptide comprises the heavy chain of factor VIIa fused to a CL domain of an antibody; and wherein the first fusion polypeptide and the second fusion polypeptide are expressed in host cells. In certain embodiments, the heteromer is secreted by the host cells.

In certain embodiments, the heteromer according to the present invention is present in a pharmaceutical composition. The pharmaceutical composition may include further pharmaceutically active agents. Alternatively, the pharmaceutical composition comprising the heteromer may be designed for use in combination with such further pharmaceutically active agents.

Furthermore, the present invention provides the heteromer according to the present invention or the pharmaceutical composition for use in medicine. Furthermore, a method for treatment comprising the administration of the heteromer according to the present invention or the pharmaceutical composition to a patient in need thereof is described. The medical use or the disease to be treated depends on the protein of interest which is formed by the heteromer.

For example, in embodiments wherein the protein of interest is an activated blood coagulation factor or the first and/or second protein of interest is a blood coagulation factor, the medical use is in the treatment of blood clotting diseases. In particular, a heteromer wherein the protein of interest is factor VIIa or another activated blood coagulation factor, or wherein the first protein of interest or the second protein of interest is factor VII or another blood coagulation factor, can be used in the treatment of a disease selected from the group consisting of hemophilia A and B; acquired hemophilia; congenital factor VII-deficiency; hemophilia prophylaxis for patients with inhibitors, trauma, bleeding in emergencies or surgical intervention such as spinal and cardiac surgery; Glanzmann's thrombasthenia; blast lung injury; and hemorrhages such as intracerebral hemorrhage, unspecific hemorrhage, or diffuse alveolar hemorrhage. Especially, the disease is hemophilia A or B, in particular in patients with inhibitors, factor VII deficiency or Glanzmann's thrombasthenia.

In another embodiment, wherein the protein of interest or at least one of the first and the second protein of interest is a gonadotropin, in particular follicle stimulating hormone, the medical use is in infertility treatment. The infertility treatment includes assisted reproductive technologies, ovulation induction, in-vitro fertilization, for example in-vitro fertilization with intracytoplasmic sperm injection, gamete intrafallopian transfer, intrauterine insemination, treatment of anovulatory disorder in women, treatment of severe hormone deficiency disorder for egg maturation in woman, treatment of sperm production deficiencies in men, and/or the enablement or improvement of germ cell maturation such as folliculogenesis and spermatogenesis, in particular follicle maturation in women, for example during in vitro fertilization stimulation protocols and/or for anovulatory disorder treatment.

The heteromer may be administered to the patient using any suitable administration route. In specific embodiments, the heteromer is administered parenterally, in particular by injection or infusion, for example intravenously, intramuscularly or subcutaneously. In further embodiments, the heteromer may also be administered by inhalation.

Also described are homodimers of one of the fusion polypeptides described herein. In particular, in the absence of the second fusion polypeptide, also the CH1 domains of two first fusion polypeptides or the CL domains of two second fusion polypeptides as described herein can bind to each other, forming a homodimer comprising two first fusion polypeptides or two second fusion polypeptides. The features, embodiments and examples described above with respect to the heteromers likewise also apply to the homodimers.

In particular, a homodimer comprising two fusion polypeptides is provided, wherein each fusion polypeptide comprises a protein of interest or a portion thereof fused to a CL domain of an antibody. The CL domain in particular comprises the amino acid sequence of SEQ ID NO: 4 or a sequence derived therefrom. Alternatively, a homodimer comprising two fusion polypeptides is provided, wherein each fusion polypeptide comprises a protein of interest or a portion thereof fused to a CH1 domain of an antibody. The fusion polypeptide may comprise further fusion partners as described herein, in particular fusion partners which elongate the circulation half-life of the homodimer such as serum albumin.

The protein of interest may be any polypeptide as described herein. In particular, it is a protein which is derived from a natural protein which is present in nature as homodimer. In this embodiment, the fusion polypeptide in particular comprises the protein of interest of a functional part thereof. Furthermore, the protein of interest may be derived from a natural protein which comprises two identical portions or subunits. In this embodiment, the fusion polypeptide in particular comprises said portion or subunit of the protein of interest of a functional part thereof.

In specific embodiments, the protein of interest present in the homodimer exhibits one or more of the properties, in particular biological and/or enzymatic activities and/or binding affinities and/or specificities, of the natural protein from which it is derived. This includes embodiments wherein the properties of the protein of interest in the homodimer are lower than in the natural protein from which it is derived. For example, the protein of interest has at least 1%, in particular at least 10% or at least 25% of the biological or enzymatic activity or binding affinity or specificity of the natural protein.

Also described are nucleic acids encoding the fusion polypeptide of the homodimer, expression cassettes and vectors comprising said nucleic acid, host cells comprising said expression cassette or vector, methods of producing the homodimer using said host cells, pharmaceutical compositions comprising the homodimer, and the homodimer for use in medicine. The features, embodiments and examples described herein for the respective aspects concerning the heteromer also likewise apply to the aspects concerning the homodimer.

Numeric ranges described herein are inclusive of the numbers defining the range. The headings provided herein are not limitations of the various aspects or embodiments of this invention which can be read by reference to the specification as a whole. According to one embodiment, subject matter described herein as comprising certain steps in the case of methods or as comprising certain ingredients in the case of compositions refers to subject matter consisting of the respective steps or ingredients. It is preferred to select and combine specific aspects and embodiments described herein and the specific subject-matter arising from a respective combination of specific embodiments also belongs to the present disclosure.

### FIGURES

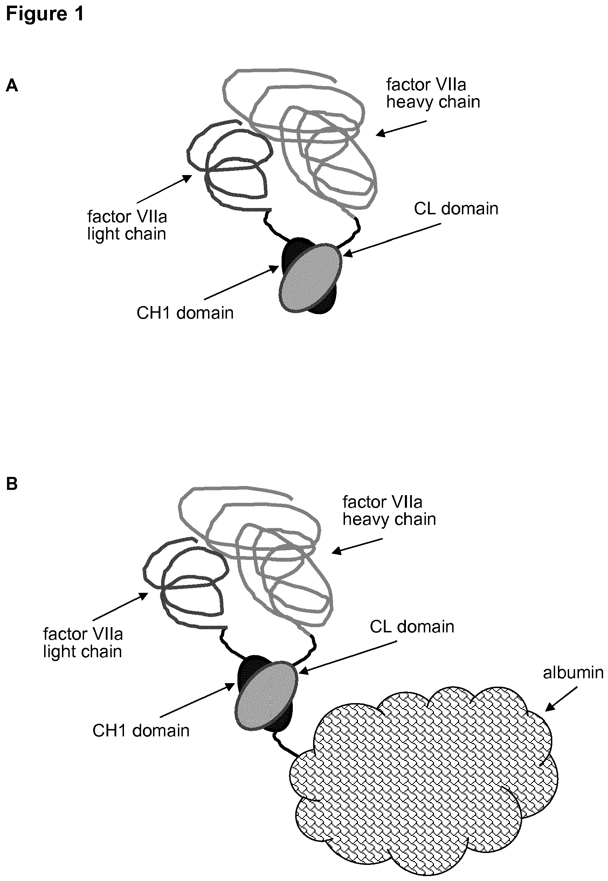

**Figure 1** shows a schematic drawing of a heteromer comprising a first polypeptide comprising, from N to C terminus, the light chain of factor VIIa, a CH1 domain, and serum albumin (only (B)), and a second polypeptide comprising, from N to C terminus, the heavy chain of factor VIIa and a CL domain.

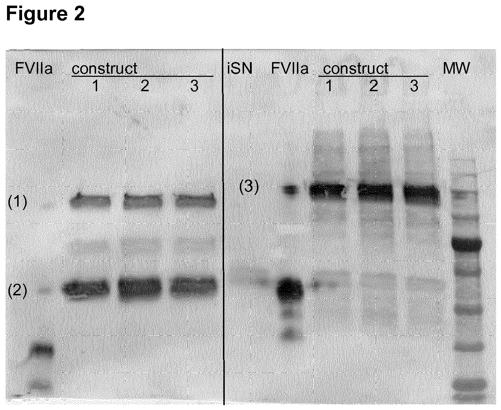

**Figure 2** shows Western Blot analysis of three factor VIIa albumin constructs (see Example 2). FVIIa, iSN: positive and negative controls, respectively; MW: molecular weight marker; Constructs 1, 2, 3: tested constructs; left: reduced conditions, resulting in dissociation of the expressed proteins to (1) factor VIIa light chain fused to CH1 domain and albumin, and (2) factor VIIa heavy chain fused to CL domain; right: non-reduced conditions, expressed dimeric proteins remain associated (3).

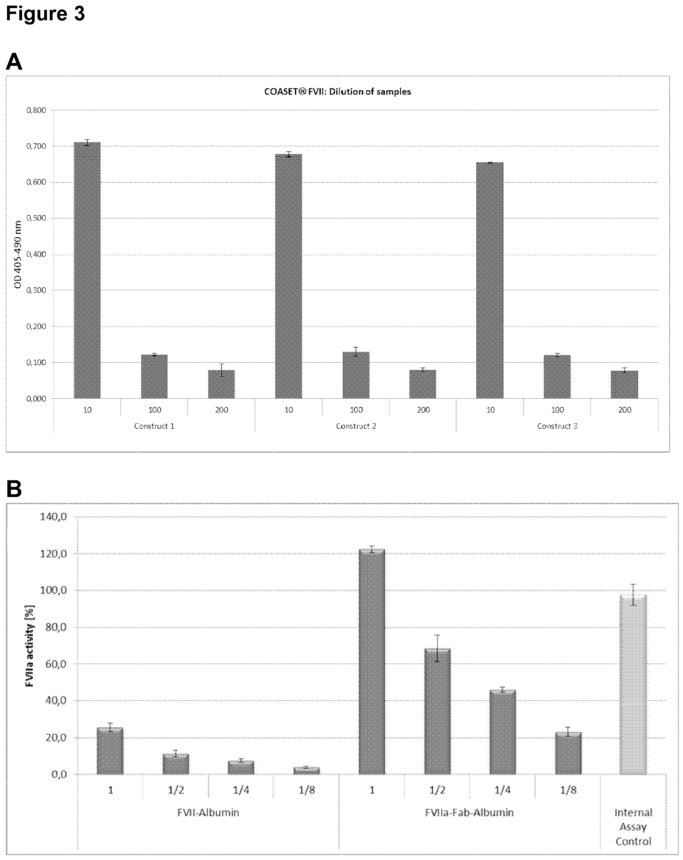

**Figure 3** shows (A) proteolytic activity of factor VIIa heterodimeric albumin constructs 1, 2 and 3 determined by the chromogenic COASET® FVII assay at three different dilutions; and (B) thrombin activation of a factor VIIa heterodimeric albumin construct in comparison to a factor VII albumin single chain construct (see Example 3).

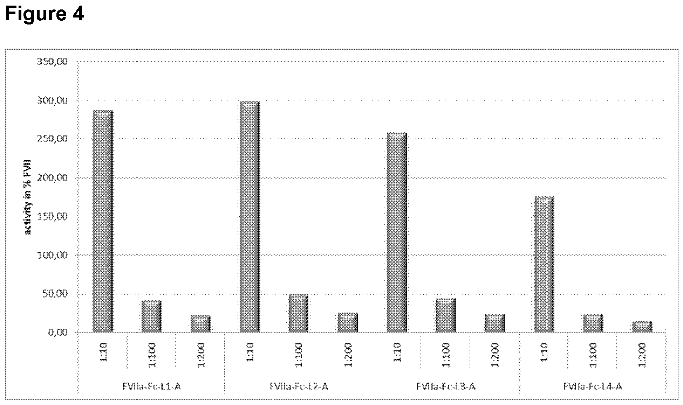

**Figure 4** shows proteolytic activity of factor VIIa heterodimeric Fc tail containing constructs determined by the chromogenic COASET® FVII assay at three different dilutions (see Example 4).

### EXAMPLES

### Example 1: Production of factor VIIa as CH1/CL fusion constructs

DNA of the factor VIIa light chain was cloned to a CH1 domain of an antibody and DNA of the factor VIIa heavy chain was cloned to a CL domain (Ckappa) of the antibody. Three different versions of the CH1 domain were used, which each comprised a different additional C terminal sequence derived from the hinge region which comprise a cysteine residue for formation of the disulfide bridge with the CL domain (EPKSC, EPKSCDK, or EPKSCDKTHT). The construct resulting from cloning the factor VIIa heavy chain to the CL domain was designated as "DNA 1", the constructs resulting from cloning the factor VIIa light chain to the CH1 domain were additionally cloned upstream to the DNA of albumin and were designated as "DNA 2".

In a next step, DNA 1 was cloned into one of Glycotope's expression vectors and DNA 2, was cloned into a second expression vector, each vector containing a resistance marker gene different from the other.

Combinations of expression vectors containing DNA 1 and DNA 2 were used for transfection of GEX™ cell lines for expression and assembly of the following constructs:
- Construct 1 = factor VIIa light chain fused to CH1 including the sequence EPKSC and to albumin; factor VIIa heavy chain fused to CL domain;
- Construct 2 = factor VIIa light chain fused to CH1 including the sequence EPKSCDK, and to albumin; factor VIIa heavy chain fused to CL domain;
- Construct 3 = factor VIIa light chain fused to CH1 including the sequence EPKSCDKTHT and to albumin; factor VIIa heavy chain fused to CL domain.

Selection pressure was applied to the cell culture and titers of factor VIIa were controlled by ELISA. Afterwards, expression of correct constructs was analyzed by Western Blot (see Example 2). Activity of constructs was measured by a chromogenic assay (see Example 3). Proteins were purified by affinity chromatography.

### Example 2: Expression control by Western Blot

To control for expression of the correct constructs and dimer assembly, cell culture supernatant was separated by SDS-PAGE under reduced (Figure 2, lanes 1-4) and non-reduced (Figure 2, lanes 5-9) conditions. After blotting and blocking of the membrane, factor VIIa was detected by an anti- factor VII primary antibody, which was in turn detected by an AP-conjugated secondary antibody. As controls, irrelevant cell culture supernatant (iSN; negative control) and factor VIIa without fusion proteins (FVIIa, positive control) were used. As can be seen from Figure 2, under reduced conditions the expressed proteins dissociate to (1) factor VIIa light chain fused to CH1 domain and albumin and (2) factor VIIa heavy chain fused to CL domain. Under non-reduced conditions, the associated heterodimeric proteins are visible (3).

The results demonstrate that the constructs comprising factor VIIa light or heavy chains were expressed and expression resulted in association via dimerization of CH1 and CL domains.

### Example 3: Activity of the factor VIIa CH1/CL fusion constructs

Activities of factor VIIa heterodimeric albumin constructs prepared according to Example 1 were determined by the chromogenic COASET® FVII assay following the instructions of the manufacturer. In this assay, factor X is activated to factor Xa by factor VIIa. Subsequently, factor Xa separates the chromophoric group pNA from a substrate (S-2765) that can be measured photometrically at 405 nm. Accordingly, the assay provides a readout for the activities of the factor VIIa heterodimeric albumin constructs. The principle of the assay is schematically depicted below:

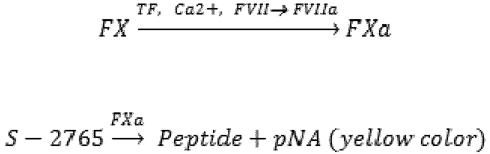

The results are shown in Figure 3A. Activity of the prepared constructs was tested using three different dilutions for each sample.

As can be seen from Figure 3A, a strong color signal was obtained for each of the constructs, indicating that the constructs are biologically active. Results tended to be particularly good for Construct 1 with CH1 including sequence EPKSC.

Furthermore, factor VII activity of the heterodimeric factor VIIa construct in comparison to an factor VII albumin fusion construct (a single chain construct comprising non-activated factor VII fused to the N terminus of albumin) was measured using the Quick assay, performed on the fully automated coagulation analyzer Ceveron® alpha TGA (Technoclone, Austria). In principle, plasma is mixed with TF and phospholipid at 37°C. Coagulation is initiated by addition of calcium chloride. The time taken from the addition of calcium to the formation of the fibrin clot is known as the prothrombin time. Factor VII samples were diluted in PBS/10% factor VII deficient plasma and the PT-assay was run using citrate buffer (Technoclone), Technoplastin HIS (Technoclone), factor VII deficient plasma (American diagnostica) according to the manufacturer's protocol. Factor VII activity was calculated using the internal calibration of the coagulation analyzer. The results are shown in Figure 3B and demonstrate that the heterodimeric factor VIIa constructs according to the invention are much more active than conventional factor VII constructs.

### Example 4: Activity of the factor Vila CH1/CL fusion constructs containing an Fc tail

Heteromer constructs wherein the CH1 fusion polypeptide comprises an Fc tail instead of an albumin were prepared and analyzed according to the above examples. Herein, a hinge-CH2-CH3 polypeptide was C terminally attached to the CH1 domain and a third polypeptide comprising another hinge-CH2-CH3 domain structure was also expressed. Due to specific mutations in the CH2 and CH3 domains, a "free" hinge-CH2-CH3 structure always pairs with a hinge-CH2-CH3 structure carrying the factor VIIa light chain - CH1 structure. Four different constructs with varying linker lengths between the CH1/CL domain and the factor VIIa part were tested in the chromogenic COASET® FVII assay. As shown in Figure 4, all constructs displayed strong biologically activity.

### Example 5: Recovery the factor VIIa CH1/CL fusion construct with albumin in the DSP process

DSP processes of the factor VII proprotein and the factor VIIa CH1/CL fusion construct with albumin were established with the same chromatography materials and in the same order. In comparison of the recoveries the second step is more efficient for the preactivated fusion construct.

The factor VII proprotein has to be activated to obtain the functional (means proteolytically active) protein. The activation of factor VII resulting in a light and a heavy chain linked via a disulfide bridge is facilitated by factor VII itself in an autoproteolytic manner during the downstream processing (DSP). In case of the preactivated fusion construct, the light chain and the heavy chain were translated separately in different peptides. The functionally active factor VIIa fusion protein is formed in the cell and secreted in the supernatant. An activation step is not necessary for this construct.

| **Step of DSP process** | **Recovery [%]** | |
|---|---|---|
| | factor VII | factor VIIa fusion |
| VIISelect | 100 | 100 |
| Capto adhere ImpRes | 38 | 77 |
| Crossflow | 78 | 90 |
| total | 29 | 69 |

As demonstrated, the overall recovery of the fusion protein according to the present invention is more than double of that of the standard protein.

## Claims

1. A heteromer comprising a first fusion polypeptide and a second fusion polypeptide, wherein the first fusion polypeptide comprises a first portion of a protein of interest fused to a CH1 domain of an antibody, and the second fusion polypeptide comprises a second portion of the protein of interest fused to a CL domain of an antibody; wherein the protein of interest is an activated blood coagulation factor.

2. The heteromer according to claim 1, wherein the first portion and the second portion form said protein of interest in the heteromer.

3. The heteromer according to claim 1 or 2, wherein the protein of interest is selected from the group consisting of fibrin, thrombin, factor Va, factor VIIa, factor VIIIa, factor IXa, factor Xa, factor XIa, factor XIIa and factor XIIIa.

4. The heteromer according to any one of claims 1 to 3, wherein the first portion is the light chain of the activated blood coagulation factor and the second portion is the heavy chain of the activated blood coagulation factor; or the first portion is the heavy chain of the activated blood coagulation factor and the second portion is the light chain of the activated blood coagulation factor.

5. The heteromer according to any one of claims 1 to 4, wherein the protein of interest is factor VIIa and the first portion is the light chain of factor VIIa and the second portion is the heavy chain of factor VIIa; or the first portion is the heavy chain of factor VIIa and the second portion is the light chain of factor VIIa.

6. The heteromer according to any one of claims 1 to 5, wherein the CH1 domain is fused to the C terminus of the first portion or first protein of interest, respectively, and/or the CL domain is fused to the C terminus of the second portion or second protein of interest, respectively.

7. The heteromer according to any one of claims 1 to 6, wherein a linker sequence is present between the first portion or first protein of interest, respectively, and the CH1 domain and/or between the second portion or second protein of interest, respectively, and the CL domain.

8. The heteromer according to any one of claims 1 to 7, wherein the first fusion polypeptide and/or the second fusion polypeptide comprises a further fusion partner.

9. The heteromer according to claim 8, wherein the further fusion partner is selected from the group consisting of albumin, the hinge, CH2 and CH3 portion of an antibody, elastin-like polypeptides, albumin binding domains, His-tag and glutathione-S-transferase (GST) tag.

10. The heteromer according to claim 8, wherein the further fusion partner does not comprise a CH2 domain and/or a CH3 domain of an antibody.

11. The heteromer according to any one of claims 1 to 10, wherein the CH1 domain is derived from an IgG antibody, in particular an IgG1 antibody, and/or wherein the CL domain is derived from a Cκ light chain.

12. The heteromer according to any one of claims 1 to 11 for use in medicine.

13. A nucleic acid encoding the first fusion polypeptide and/or the second fusion polypeptide as defined in any one of claims 1 to 11.

14. A host cell comprising the nucleic acid according to claim 13.

15. A method of producing the heteromer according to any one of claims 1 to 14, wherein the first fusion polypeptide and the second fusion polypeptide are expressed in host cells.

16. The method according to claim 15, wherein the first fusion polypeptide and the second fusion polypeptide are co-expressed in the same host cell, and/or are secreted by the host cells.

## Patentansprüche

1. Heteromer umfassend ein erstes Fusionspolypeptid und ein zweites Fusionspolypeptid, wobei das erste Fusionspolypeptid einen ersten Teil eines Proteins von Interesse umfasst, welcher mit einer CH1-Domäne eines Antikörpers fusioniert ist, und das zweite Fusionspolypeptid einen zweiten Teil des Proteins von Interesse umfasst, welcher mit einer CL-Domäne eines Antikörpers fusioniert ist, wobei das Protein von Interesse ein aktivierter Blutkoagulationsfaktor ist.

2. Das Heteromer nach Anspruch 1, wobei der erste Teil und der zweite Teil das Protein von Interesse im Heteromer bilden.

3. Das Heteromer nach Anspruch 1 oder 2, wobei das Protein von Interesse ausgewählt ist aus der Gruppe bestehend aus Fibrin, Thrombin, Faktor Va, Faktor VIIa, Faktor VIIIa, Faktor IXa, Faktor Xa, Faktor XIa, Faktor XIIa und Faktor XIIIa.

4. Das Heteromer nach einem der Ansprüche 1 bis 3, wobei der erste Teil die leichte Kette des aktivierten Blutkoagulationsfaktors und der zweite Teil die schwere Kette des aktivierten Blutkoagulationsfaktors ist; oder der erste Teil die schwere Kette des aktivierten Blutkoagulationsfaktors und der zweite Teil die leichte Kette des aktivierten Blutkoagulationsfaktors ist.

5. Das Heteromer nach einem der Ansprüche 1 bis 4, wobei das Protein von Interesse Faktor VIIa ist und der erste Teil die leichte Kette des Faktors VIIa ist und der zweite Teil die schwere Kette des Faktors VIIa ist; oder der erste Teil die schwere Kette des Faktors VIIa und der zweite Teil die leichte Kette des Faktors VIIa ist.

6. Das Heteromer nach einem der Ansprüche 1 bis 5, wobei die CH1-Domäne mit dem C-Terminus des ersten Teils bzw. des ersten Proteins von Interesse fusioniert ist, und/oder die CL-Domäne mit dem C-Terminus des zweiten Teils bzw. des zweiten Proteins von Interesse fusioniert ist

7. Das Heteromer nach einem der Ansprüche 1 bis 6, wobei eine Linkersequenz zwischen dem ersten Teil bzw. dem ersten Protein von Interesse und der CH1-Domäne und/oder zwischen dem zweiten Teil bzw. zweiten Protein von Interesse und der CL-Domäne vorhanden ist.

8. Das Heteromer nach einem der Ansprüche 1 bis 7, wobei das erste Fusionspolypeptid und/oder das zweite Fusionspolypeptid einen weiteren Fusionspartner beinhalten.

9. Das Heteromer nach Anspruch 8, wobei der weitere Fusionspartner ausgewählt ist aus der Gruppe bestehend aus Albumin, dem Scharnier-, CH2- und CH3-Teil eines Antikörpers, Elastin-ähnliche Polypeptide, Albumin-Bindungsdomänen, His-Tag und Glutathion-S-Transferasen (GST) Tag.

10. Das Heteromer nach Anspruch 8, wobei der weitere Fusionspartner keine CH2-Domäne und/oder CH3-Domäne eines Antikörpers aufweist.

11. Das Heteromer nach einem der Ansprüche 1 bis 10, wobei sich die CH1-Domäne von einem IgG Antikörper, besonders von einem IgG1 Antikörper, ableitet, und/oder wobei die CL-Domäne sich von einer leichten Cκ-Kette ableitet.

12. Das Heteromer nach einem der Ansprüche 1 bis 11 zur Verwendung in der Medizin.

13. Nukleinsäure, die das erste Fusionspolypeptid und/oder das zweite Fusionspolypeptid nach einem der Ansprüche 1 bis 11 kodiert.

14. Wirtszelle, umfassend eine Nukleinsäure nach Anspruch 13.

15. Das Verfahren zur Herstellung des Heteromers nach einem der Ansprüche 1 bis 14, wobei das erste Fusionspolypeptid und das zweite Fusionspolypeptid in einer Wirtszelle exprimiert werden.

16. Das Verfahren nach Anspruch 15, wobei das erste Fusionspolypeptid und das zweite Fusionspolypeptid in derselben Wirtszelle co-expressioniert werden, und/oder von der Wirtszelle sekretiert werden.

## Revendications

1. Hétéromère comprenant un premier polypeptide de fusion et un second polypeptide de fusion, dans lequel le premier polypeptide de fusion comprend une première portion d'une protéine pertinente fusionnée à un domaine CH1 d'un anticorps, et le second polypeptide de fusion comprend une seconde portion de la protéine pertinente fusionnée à un domaine CL d'un anticorps ; dans lequel la protéine pertinente est un facteur de coagulation sanguine activé.

2. Hétéromère selon la revendication 1, dans lequel la première portion et la seconde portion forment ladite protéine pertinente dans l'hétéromère.

3. Hétéromère selon la revendication 1 ou 2, dans lequel la protéine pertinente est sélectionnée parmi le groupe constitué de la fibrine, thrombine, du facteur Va, facteur VIIa, facteur VIIIa, facteur IXa, facteur Xa, facteur XIa, facteur XIIa et facteur XIIIa.

4. Hétéromère selon l'une quelconque des revendications 1 à 3, dans lequel la première portion est la chaîne légère du facteur de coagulation sanguine activé et la seconde portion est la chaîne lourde du facteur de coagulation sanguine activé ; ou la première portion est la chaîne lourde du facteur de coagulation sanguine activé et la seconde portion est la chaîne légère du facteur de coagulation sanguine activé.

5. Hétéromère selon l'une quelconque des revendications 1 à 4, dans lequel la protéine pertinente est le facteur VIIa et la première portion est la chaîne légère du facteur VIIa et la seconde portion est la chaîne lourde du facteur VIIa ; ou la première portion est la chaîne lourde du facteur VIIa et la seconde portion est la chaîne légère du facteur VIIa.

6. Hétéromère selon l'une quelconque des revendications 1 à 5, dans lequel le domaine CH1 est fusionné à la terminaison C de la première portion ou première protéine pertinente, respectivement, et/ou le domaine CL est fusionné à la terminaison C de la seconde portion ou seconde protéine pertinente, respectivement.

7. Hétéromère selon l'une quelconque des revendications 1 à 6, dans lequel une séquence liante est présente entre la première portion ou première protéine pertinente, respectivement, et le domaine CH1 et/ou entre la seconde portion ou seconde protéine pertinente, respectivement, et le domaine CL.

8. Hétéromère selon l'une quelconque des revendications 1 à 7, dans lequel le premier polypeptide de fusion et/ou le second polypeptide de fusion comprend un autre partenaire de fusion.

9. Hétéromère selon la revendication 8, dans lequel l'autre partenaire de fusion est sélectionné parmi le groupe constitué de l'albumine, la portion charnière, CH2 et CH3 d'un anticorps, des polypeptides de type élastine, des domaines de liaison à l'albumine, His-tag (étiquette poly-histidine) et d'une étiquette glutathion-S-transférase (GST).

10. Hétéromère selon la revendication 8, dans lequel l'autre partenaire de fusion ne comprend pas de domaine CH2 et/ou de domaine CH3 d'un anticorps.

11. Hétéromère selon l'une quelconque des revendications 1 à 10, dans lequel le domaine CH1 est dérivé d'un anticorps IgG, notamment d'un anticorps IgG1, et/ou dans lequel le domaine CL est dérivé d'une chaîne légère Cκ.

12. Hétéromère selon l'une quelconque des revendications 1 à 11 destiné à être utilisé en médecine.

13. Acide nucléique codant pour le premier polypeptide de fusion et/ou le second polypeptide de fusion tels que définis dans l'une quelconque des revendications 1 à 11.

14. Cellule hôte comprenant l'acide nucléique selon la revendication 13.

15. Procédé de production de l'hétéromère selon l'une quelconque des revendications 1 à 14, dans lequel le premier polypeptide de fusion et le second polypeptide de fusion sont exprimés dans des cellules hôtes.

16. Procédé selon la revendication 15, dans lequel le premier polypeptide de fusion et le second polypeptide de fusion sont co-exprimés dans la même cellule hôte, et/ou sont secrétés par les cellules hôtes.


### SEQUENCE LISTING

<110> Glycotope GmbH
<120> Heteromers comprising antibody domain fusion proteins
<130> 57 300 K
<150> LU 92791
   <151> 2015-08-06
<150> EP 15 180 252.7
   <151> 2015-08-07
<160> 7
<170> PatentIn version 3.5
<210> 1
   <211> 5
   <212> PRT
   <213> Artificial Sequence
<220>
   <223> amino acid linker
<400> 1
   Gly Gly Gly Gly Ser
   1               5
<210> 2
   <211> 98
   <212> PRT
   <213> Homo sapiens
<400> 2
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val
<210> 3
   <211> 10
   <212> PRT
   <213> Homo sapiens
<400> 3
   Glu Pro Lys Ser Cys Asp Lys Thr His Thr
   1 5 10
<210> 4
   <211> 106
   <212> PRT
   <213> Homo sapiens
<400> 4
   Thr Val Ala Ala Pro Ser Val Phe Ile Phe Pro Pro Ser Asp Glu Gln
   1               5                   10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu Cys
               100                 105
<210> 5
   <211> 106
   <212> PRT
   <213> Homo sapiens
<400> 5
   Gly Gln Pro Lys Ala Asn Pro Thr Val Thr Leu Phe Pro Pro Ser Ser
   1               5                   10                  15
   Glu Glu Leu Gln Ala Asn Lys Ala Thr Leu Val Cys Leu Ile Ser Asp
               20                  25                  30
   Phe Tyr Pro Gly Ala Val Thr Val Ala Trp Lys Ala Asp Gly Ser Pro
           35                  40                  45
   Val Lys Ala Gly Val Glu Thr Thr Lys Pro Ser Lys Gln Ser Asn Asn
       50                  55                  60
   Lys Tyr Ala Ala Ser Ser Tyr Leu Ser Leu Thr Pro Glu Gln Trp Lys
   65                  70                  75                  80
   Ser His Arg Ser Tyr Ser Cys Gln Val Thr His Glu Gly Ser Thr Val
                   85                  90                  95
   Glu Lys Thr Val Ala Pro Thr Glu Cys Ser
               100                 105
<210> 6
   <211> 106
   <212> PRT
   <213> Homo sapiens
<400> 6
   Gly Gln Pro Lys Ala Ala Pro Ser Val Thr Leu Phe Pro Pro Ser Ser
   1               5                   10                  15
   Glu Glu Leu Gln Ala Asn Lys Ala Thr Leu Val Cys Leu Ile Ser Asp
               20                  25                  30
   Phe Tyr Pro Gly Ala Val Thr Val Ala Trp Lys Ala Asp Ser Ser Pro
           35                  40                  45
   Val Lys Ala Gly Val Glu Thr Thr Thr Pro Ser Lys Gln Ser Asn Asn
       50                  55                  60
   Lys Tyr Ala Ala Ser Ser Tyr Leu Ser Leu Thr Pro Glu Gln Trp Lys
   65                  70                  75                  80
   Ser His Arg Ser Tyr Ser Cys Gln Val Thr His Glu Gly Ser Thr Val
                   85                  90                  95
   Glu Lys Thr Val Ala Pro Thr Glu Cys Ser
               100                 105
<210> 7
   <211> 466
   <212> PRT
   <213> Homo sapiens
<400> 7